# FASCICULE DE BREVET EUROPEEN

(11) **EP 3 289 068 B1**
(45) Date de publication et mention de la délivrance du brevet: **14.12.2022**
(21) Numéro de dépôt: 16722317.1
(22) Date de dépôt: 27.04.2016
(51) Int. Cl.: C12N 1/18, C12N 1/19, C12N 1/36, C12P 7/10, C12R 1/865

(54) **SOUCHE DE LEVURE PRESENTANT UNE CAPACITE AMELIOREE A FERMENTER LE XYLOSE EN PRESENCE D'ACIDE ACETIQUE**
HEFESTAMM MIT VERBESSERTER FÄHIGKEIT FÜR DIE FERMENTATION VON XYLOSE IN GEGENWART EINER ESSIGSÄURE
YEAST STRAIN HAVING IMPROVED CAPABILITY FOR FERMENTING XYLOSE IN THE PRESENCE OF ACETIC ACID

(30) Priorité: 27.04.2015 FR 1553760
(43) Date de publication de la demande: 07.03.2018
(73) Titulaire: Lesaffre et Compagnie, 75001 Paris (FR)
(72) Inventeur: BAVOUZET, Jean-Michel, 59170 Croix (FR); DESFOUGERES, Thomas, 86130 Dissay (FR); PIGNEDE, Georges, 59700 Marcq-en-baroeul (FR); TECHEL, Jennifer, 7783 Le Bizet (BE)
(74) Mandataire: Cabinet Laurent & Charras
(86) Numéro de dépôt international: PCT/FR2016/050987
(87) Numéro de publication internationale: WO 2016/174349

(56) Documents cités:
- WO-A1-2013/178915
- US-A1- 2011 159 560
- JEREMIAH WRIGHT ET AL: "Batch and continuous culture-based selection strategies for acetic acid tolerance in xylose-fermenting Saccharomyces cerevisiae", FEMS YEAST RESEARCH, vol. 11, no. 3, 1 mai 2011 (2011-05-01), pages 299-306, XP055202939, ISSN: 1567-1356, DOI: 10.1111/j.1567-1364.2011.00719.x

## Description

### DOMAINE DE L'INVENTION

La présente invention a trait aux souches de levures aptes à fermenter les pentoses, en particulier le xylose, y compris en présence d'inhibiteurs de cette fermentation tels que l'acide acétique sous forme non dissociée.

Plus précisément, la présente invention offre un procédé permettant de sélectionner des souches améliorées, très performantes dans leur capacité à métaboliser ce type de sucres présents dans les hydrolysats lignocellulosiques.

### ETAT DE LA TECHNIQUE

La biomasse lignocellulosique ou végétale, issue essentiellement de l'activité agricole et agro-industrielle, est un substrat complexe, constitué de trois principales fractions que sont la cellulose, les hémicelluloses et la lignine. Il s'agit d'un déchet potentiellement recyclable, utile pour la production d'éthanol dont la demande, en vue par exemple de son utilisation en tant que biocarburant, ne cesse de croitre.

Le procédé de production d'éthanol à partir de la biomasse lignocellulosique consiste à récupérer par hydrolyse le maximum de sucres présents dans les fractions cellulosiques et hémicellulosiques puis de les transformer en éthanol par fermentation.

Concernant la fermentation des sucres présents dans cette biomasse comprenant à la fois des sucres en C6 (hexoses) ou des sucres en C5 (pentoses), il est aujourd'hui favorisé la fermentation anaérobie par les levures, en particulier à l'aide de *Saccharomyces cerevisiae* dont la capacité à fermenter le glucose est largement maîtrisée et exploitée.

Toutefois, toute l'attention s'est portée sur la fermentation des pentoses, notamment le xylose, qui peuvent représenter jusqu'à 25 à 40% des sucres totaux contenus dans la biomasse lignocellulosique. Ainsi, des souches de levures aptes à fermenter le glucose ont été modifiées pour pouvoir également métaboliser les pentoses.

A titre d'exemple, le document WO 2010/000464 rapporte l'obtention de souches de levure aptes à fermenter les pentoses grâce à un gène bactérien codant une xylose isomérase (XI) qui convertit le xylose en xylulose métabolisable par la levure.

A noter que de manière alternative, une voie eucaryote comprenant une xylose réductase (XR ou *XYL1)* générant du xylitol et une xylitol déshydrogénase (XDH ou *XYL2)* permet également d'aboutir au xylulose.

Ainsi, le document WO 2012/072793 décrit des souches de levure améliorées, associant des gènes exogènes codant une xylose isomérase et une xylitol déshydrogénase permettant d'éliminer le xylitol qui s'avère être un inhibiteur de la xylose isomérase. De telles souches, en particulier la souche déposée à la CNCM le 5.10.2011 sous le numéro I-4538, présentent des rendements améliorés et donc une utilité industrielle avérée pour la production d'éthanol.

Le document US 2011/159560 décrit la possibilité d'introduire des copies surnuméraires du gène *GAL2* dans une souche de levure capable de métaboliser le xylose.

Une autre problématique cruciale a été la mise en évidence, dans les hydrolysats lignocellulosiques, d'inhibiteurs de la fermentation parmi lesquels figurent des furaldéhydes (furfural, HMF), des composés phénoliques et des acides organiques (acide acétique, acide lévulinique, acide formique). En particulier, la présence de forte concentration en acide acétique, supérieure à 5 g/kg (de milieu initial) et pouvant atteindre jusqu'à 10 g/kg, est intrinsèquement liée à celle de groupements acétyles associés de manière covalente avec les molécules d'hémicellulose.

Des travaux antérieurs ont abordé l'amélioration de la résistance des souches vis-à-vis de la présence d'acide acétique dans les mouts de fermentation. Ainsi, le document WO 2011/080411 a rapporté l'obtention de souches de levure dont la résistance à l'acide acétique sur glucose a été améliorée.

Cependant, l'acide acétique est également un inhibiteur de la fermentation du xylose. Cette inhibition se caractérise par une réduction de la cinétique de consommation du xylose (Bellisimi et al., FEMS Yeast Res., 2009. 9 :358-364), alors que sur le glucose, cette inhibition se traduit par un retard lors de l'initiation de la fermentation, la cinétique restant par la suite inchangée. A noter qu'en présence à la fois de glucose et de xylose dans le milieu, les souches de levure fermentent en priorité le glucose en raison de la répression catabolique.

Ainsi, les documents WO 2013/178915 et WO 2013/178918 décrivent des procédés d'obtention de souches de levure aptes à métaboliser les pentoses, notamment le xylose, et résistantes aux inhibiteurs de fermentation, en particulier l'acide acétique.

Il existe toutefois un besoin évident d'obtenir de nouvelles souches de levure capables de fermenter les hexoses et les pentoses, avec un impact encore réduit des inhibiteurs de la fermentation, en particulier de l'acide acétique, notamment sur la cinétique de fermentation des pentoses tels que le xylose.

### DESCRIPTION DE L'INVENTION

La présente invention repose sur la mise en évidence par les inventeurs de la possibilité d'isoler de nouvelles souches de levure présentant une résistance vis-à-vis des inhibiteurs de la fermentation, en particulier de l'acide acétique, non seulement au niveau de leur métabolisme du glucose mais également au niveau de la fermentation des pentoses, en particulier du xylose.

Ainsi et dans le cadre de l'invention, il a été mis au point un procédé de sélection de souches de levure présentant une fermentation améliorée des pentoses, en particulier du xylose, en présence d'inhibiteurs de la fermentation de type acide organique, en particulier l'acide acétique. A l'aide d'un tel procédé, il a été possible d'isoler des souches présentant une cinétique de fermentation du xylose améliorée, en termes de vitesse de consommation du xylose, y compris en présence de fortes teneurs en acide acétique.

A noter que des travaux antérieurs se sont déjà intéressés à cette problématique :
Ainsi, le document (Wright et al., FEMS Yeast Res., 2011. 11: 299-306) rapporte l'amélioration de la résistance de souches vis-à-vis de l'acétate sur xylose. Ces travaux tendent à montrer que le phénomène de résistance vis-à-vis de l'acide acétique sur xylose pourrait être un processus inductible. De surcroît, les résultats présentés apparaissent comme extrêmement mitigés :
Les cultures sélectionnées par la méthode SBR (« *Single Batch Repeat* ») ou avec un chemostat limité en xylose démontrent seulement une faible diminution de la concentration finale en xylose et par voie de conséquence une faible augmentation de la concentration en éthanol au cours des 3 premiers jours, par rapport aux résultats de la souche de départ.

Par ailleurs, le pic du taux de consommation spécifique du xylose en fonction de la concentration d'acide acétique culmine pour une valeur très proche de celle observée pour la souche de départ, soit 2,5 g/L.

De plus, il est observé qu'avec la souche isolée, la vitesse de consommation spécifique du xylose décroit ensuite de façon comparable à ce qui est observé avec la souche de départ. D'après les conclusions tirées, ces résultats indiqueraient qu'une sélection prolongée en culture SBR ne peut pas conduire à un phénotype stable de tolérance à l'acide acétique.

Un autre document (Sanda et al., Bioresource Technology, 2011. 102 :7917-7924) a rapporté la mise en œuvre d'une évolution dirigée sur un hydrolysat lignocellulosique contenant du glucose et du xylose. Toutefois, la technique SBR qui permet de sélectionner les souches se multipliant le plus vite a abouti à la sélection des souches poussant le plus rapidement sur glucose.

En dépit des échecs de l'art antérieur, les inventeurs ont mis au point un nouveau procédé de sélection permettant d'aboutir à des souches de levure présentant les caractéristiques espérées en termes de fermentation du xylose en présence d'acide acétique.

Selon un premier aspect, la présente invention concerne un procédé de sélection d'une souche de levure présentant une capacité améliorée à fermenter un pentose en présence d'acide organique sous forme non dissociée,
dans lequel au moins une souche de levure présentant une capacité à fermenter ledit pentose est cultivée successivement dans les 2 milieux suivants :
   - un premier milieu de croissance comprenant comme seule source de carbone ledit pentose et ledit acide organique sous forme non dissociée ;
   - un deuxième milieu de croissance comprenant comme seule source de carbone une autre source de carbone, avantageusement du glucose, et dépourvu dudit acide organique sous forme non dissociée,
la culture successive dans au moins ces deux milieux de croissance étant répétée au moins deux fois, en présence de concentrations croissantes en acide organique sous forme non dissociée à une concentration comprise entre 1,3 et 2,6 g/L.

Ainsi, le procédé selon l'invention permet, à partir d'une souche isolée ou d'un mélange de souches, de sélectionner une souche présentant un avantage sélectif en termes de croissance sur un milieu contenant ledit pentose et ledit acide organique sous forme non dissociée.

Le procédé, objet de la présente demande, peut être mis en œuvre sur une souche isolée, en particulier sur les souches suivantes :
- la souche déposée à la CNCM en date du 16.05.2013 sous le numéro I-4749 ;
- la souche déposée à la CNCM en date du 12.12.2013 sous le numéro I-4829 ;
- la souche déposée à la CNCM en date du 9.04.2015 sous le numéro I-4966.

Dans ce cas de figure et sans vouloir être liés à une quelconque théorie, la pression de sélection exercée par les cultures successives dans les milieux de croissance définis ci-après permet à la souche d'acquérir les traits phénotypiques nécessaires à l'augmentation de sa capacité à fermenter un pentose en présence d'acide organique sous forme non dissociée.

Selon un autre aspect, c'est un mélange de souches de levure qui est soumis au procédé selon l'invention. Dans ce cas de figure, au moins l'une des souches subit la pression de sélection comme décrit ci-dessus, de manière à développer une capacité améliorée à fermenter un pentose en présence d'acide organique sous forme non dissociée. Alternativement, le mélange de souches contient déjà une souche présentant une capacité améliorée à fermenter un pentose en présence d'acide organique sous forme non dissociée et le procédé selon l'invention permet d'enrichir le mélange en cette souche, en raison de sa multiplication plus rapide, et ainsi d'isoler ou de sélectionner ladite souche.

Selon un mode de réalisation particulier, avant leur culture dans les différents milieux de croissance selon l'invention, la souche de levure ou le mélange de souches est soumis à une étape préalable de mutagénèse susceptible de favoriser l'apparition du phénotype recherché. De manière classique, cette mutagénèse peut être réalisée par exposition à un agent chimique ou à un rayonnement, notamment aux ultraviolets (UV). Il est avantageusement choisi des conditions « douces », par exemple une exposition de 100 à 500 J/cm², par exemple 300 J/cm2, de rayonnement UV à 254 nm.

Selon le procédé de l'invention, la souche de levure ou le mélange de souches est cultivé successivement dans au moins deux milieux de croissance. Dans le cadre de la présente demande, les expressions « milieu de croissance » ou « milieu de culture » sont utilisées indifféremment pour désigner un milieu comprenant les ingrédients nécessaires à la survie voire à la multiplication des levures en présence.

Le premier milieu de croissance se caractérise en ce qu'il comprend, comme seule source de carbone, le pentose dont la capacité de fermentation améliorée est recherchée. Il s'agit avantageusement du xylose ou de l'arabinose, encore plus avantageusement du xylose. De manière avantageuse, ce premier milieu est un milieu liquide.

De manière avantageuse, la concentration en pentose du milieu de croissance est celle couramment mise en œuvre lorsque celui-ci est utilisé comme seule source de carbone, à savoir comprise entre 5 et 100 g/L dans le cas d'un milieu liquide (5 et 100 g/kg dans le cas d'un milieu solide), avantageusement comprise entre 25 et 90 g/L, par exemple égale à 50 g/L. Selon un mode de réalisation particulier, le milieu de croissance contient 50 g/L de xylose.

Ledit milieu comprend également l'acide organique susceptible d'inhiber la fermentation dudit pentose. Il s'agit avantageusement d'acide acétique ou d'acide formique, encore plus avantageusement d'acide acétique.

A noter que de manière connue, seule la forme non dissociée ou non ionisée de tels acides présente une capacité d'inhibition. Dans le cadre de l'invention, on entend par « forme non ionisée ou non dissociée » d'un acide carboxylique sa forme protonée. En pratique, la forme de tels acides organiques dépend du pH du milieu dans lequel ils sont incorporés. A un pH supérieur au pKa de l'acide, celui-ci se trouve majoritairement sous forme dissociée ou d'ions COO⁻. Au contraire et à un pH inférieur, la forme majoritaire est la forme non dissociée ou non ionisée (COOH). Dans la suite de l'invention, il sera précisé, notamment en rapport avec les quantités ou concentrations, si seule la forme non dissociée de l'acide organique en présence est prise en compte, ou s'il est fait référence à l'acide acétique introduit dans le milieu, englobant formes dissociée et non dissociée en fonction du pH dudit milieu.

Selon l'invention, la concentration en acide organique sous forme non dissociée, avantageusement en acide acétique, du milieu de croissance est comprise entre 1,3 et 2,6 g/L en milieu liquide (équivalent à 1,3 et 2,6 g/kg en milieu solide). En pratique et à titre d'exemple, cette dernière fourchette correspond à une concentration en acide acétique ajoutée à un milieu de croissance à pH 5 comprise entre 3 et 6 g/L.

Selon l'invention, ce premier milieu de croissance est mis en œuvre dans un cycle de croissance sur au moins deux, voire trois milieux, ledit cycle étant répété au moins deux fois. De manière adaptée selon l'invention, au cours des au moins 2 cycles de croissance, la concentration en acide organique sous forme non dissociée est augmentée.

En d'autres termes, la culture successive dans au moins les deux milieux de croissance définis dans la cadre de l'invention est répétée au moins deux fois, en présence de concentrations croissantes en acide organique, avantageusement de l'acide acétique, sous forme non dissociée. Ainsi et en présence de n cycles (avec n supérieur ou égal à 2), au moins deux concentrations sont mises en œuvre, la première concentration étant inférieure à la seconde concentration.

A titre d'exemple et pour 8 cycles de culture en présence d'acide acétique, un schéma d'augmentation de la concentration dans le premier milieu de croissance peut être le suivant :
- 1^{er} cycle : concentration en acide acétique ajoutée dans un milieu à pH 5 égale à 3 g/L (soit 1,3 g/L d'acide acétique sous forme dissociée) ;
- 2^{ème} cycle : concentration en acide acétique ajoutée dans un milieu à pH 5 égale à 3 g/L (soit 1,3 g/L d'acide acétique sous forme non dissociée) ;
- 3^{ème} cycle : concentration en acide acétique ajoutée dans un milieu à pH 5 égale à 3 g/L (soit 1,3 g/L d'acide acétique sous forme non dissociée) ;
- 4^{ème} cycle : concentration en acide acétique ajoutée dans un milieu à pH 5 égale à 4 g/L (soit 1,7 g/L d'acide acétique sous forme non dissociée) ;
- 5^{ème} cycle : concentration en acide acétique ajoutée dans un milieu à pH 5 égale à 4 g/L (soit 1,7 g/L d'acide acétique sous forme non dissociée) ;
- 6^{ème} cycle : concentration en acide acétique ajoutée dans un milieu à pH 5 égale à 4 g/L (soit 1,7 g/L d'acide acétique sous forme non dissociée) ;
- 7^{ème} cycle : concentration en acide acétique ajoutée dans un milieu à pH 5 égale à 5 g/L (soit 2,15 g/L d'acide acétique sous forme non dissociée) ;
- 8^{ème} cycle : concentration en acide acétique ajoutée dans un milieu à pH 5 égale à 6 g/L (soit 2,6 g/L d'acide acétique sous forme non dissociée).

Outre ces deux ingrédients, ce milieu de croissance est avantageusement un milieu synthétique complet, adapté à la croissance des levures et pouvant contenir des ingrédients classiques tels que des sels, des agents tampons, de l'extrait de levure ou toute autre source d'azote métabolisable par la levure, des vitamines.... Dans le cadre de l'invention, on entend par « milieu synthétique » un milieu dont la composition chimique est connue.

Selon un mode de réalisation particulier, outre le pentose en tant que seule source de carbone et l'acide organique, un tel milieu peut donc comprendre :
- extrait de levure (EXL), avantageusement à hauteur de 5 g/L ;
- di-amonium phosphate, avantageusement à hauteur de 4,7 g/L ;
- acide citrique, avantageusement à hauteur de 11,4 g/L ;
- citrate tri-sodique, avantageusement à hauteur de 13,5 g/L ;
- ZnSO₄, avantageusement à hauteur de 21,2 mg/L ;
- MgSO₄ 7H₂O, avantageusement à hauteur de 1 g/L ;
- Thiamine, avantageusement à hauteur de 18,24 mg/L ;
- Pyridoxine, avantageusement à hauteur de 5,28 mg/L ;
- Biotine, avantageusement à hauteur de 1,76 g/L ;
- Panthoténate, avantageusement à hauteur de 3,8 mg/L ;
- acide nitotinique, avantageusement à hauteur de 20 mg/L ;
- myo-inositol, avantageusement à hauteur de 50 mg/L ;
- riboflavine, avantageusement à hauteur de 1 mg/L ;
- para-amino-benzoate, avantageusement à hauteur de 1,2 mg/L ;
- tween 80, avantageusement à hauteur de 1 g/L.

Outre la composition spécifique de ce premier milieu de croissance, la culture de la souche de levure ou du mélange de souches est avantageusement réalisée dans des conditions standard favorables à la croissance des levures, en particulier de type *Saccharomyces,* et à leur activité de fermentation, à savoir :
- un pH acide, avantageusement compris entre 4 et 6, voire 4,5 et 5,5, encore plus avantageusement égal à 5 ;
- une température comprise entre 28 et 37°C, voire entre 30 et 35°C, avantageusement égale à 32°C ;
- sous faible agitation, par exemple égale à 100 rpm ;
- dans des conditions réduites d'apport en oxygène (sous O₂ limité). En pratique, la culture peut être réalisée dans une fiole obturée à l'aide d'un bouchon réduisant l'apport d'O₂ dans le milieu tout en permettant l'évacuation du CO₂ produit.

De manière générale, la culture est stoppée lorsque la source de carbone osidique, en l'occurrence le pentose, a été totalement consommée. En pratique et de manière avantageuse, la culture est menée pendant au moins 24 heures, voire plusieurs jours, avantageusement 7 jours.

La deuxième étape du procédé selon l'invention consiste à transférer les levures cultivées dans le premier milieu de croissance dans un deuxième milieu de croissance, avantageusement liquide. De manière caractéristique, celui-ci se distingue du premier milieu de culture par la présence d'une source de carbone différente et par l'absence de l'acide organique, avantageusement de l'acide acétique, sous forme non dissociée.

En pratique, ce milieu est favorable à la croissance des levures et permet de lever les phénomènes d'adaptation, au profit de l'acquisition de mutations stables. Ainsi, cette étape du procédé selon l'invention est considérée comme une phase de désadaptation.

Ce second milieu de croissance se caractérise en ce qu'il comprend, comme seule source de carbone, une source de carbone différente de celle du premier milieu de culture, à savoir un pentose, en particulier du xylose. Selon un mode de réalisation particulier la source de carbone du second milieu de culture est une source de carbone osidique, avantageusement un hexose, encore plus avantageusement du glucose. De manière avantageuse, la concentration en cette seconde source de carbone, avantageusement en glucose, est comprise entre 5 et 50 g/L, avantageusement comprise entre 5 et 20 g/L, par exemple égale à 20 g/L.

Par ailleurs et de manière avantageuse, il s'agit d'un milieu de croissance synthétique riche, comprenant par exemple :
- de l'extrait de levure, avantageusement à hauteur de 10 g/L ;
- de la peptone, avantageusement à hauteur de 10 g/L .

De manière avantageuse, il s'agit donc d'un milieu riche permettant à toutes les souches de levures de se multiplier sans aucune limitation qui serait liée à une quelconque auxotrophie.

Outre la composition spécifique de ce second milieu de croissance, la culture de la souche de levure ou du mélange de souches est avantageusement réalisée dans des conditions standard favorables à la croissance des levures, à savoir :
- un pH acide et stable, avantageusement compris entre 4 et 6, par exemple égal à 5 ;
- une température comprise entre 28 et 37°C, voire entre 30 et 35°C, avantageusement égale à 30°C ;
- sous agitation moyenne, par exemple égale à 150 rpm ;
- en présence d'oxygène, à savoir en aérobiose. En pratique, la culture peut être réalisée dans une fiole à baffles obturée par un bouchon poreux qui permet l'apport d'O₂ dans le milieu et l'évacuation du CO₂ produit.

Là encore, la culture est stoppée lorsque la source de carbone osidique, avantageusement le glucose, a été totalement consommée. Il est à noter qu'en pratique, la croissance dans ce second milieu est plus rapide que dans le premier milieu en raison de l'aérobiose, du glucose en tant que source de carbone et de l'absence d'acide acétique. Ainsi et de manière avantageuse, la culture est menée pendant plusieurs heures, avantageusement 24 heures.

Selon l'invention, la culture des levures successivement dans les deux milieux de croissance et dans les conditions décrites ci-dessus constitue un cycle.

Selon un mode de réalisation particulier, un cycle dans le procédé selon l'invention comprend en outre le passage des levures dans un troisième milieu de croissance, avantageusement liquide, destiné à sélectionner les cellules capables de respirer, à savoir présentant des mitochondries fonctionnelles. En pratique, cette étape, qui peut être mise en œuvre à chaque cycle ou au moins une fois dans le procédé, permet de s'affranchir de l'apparition des « petites », dont le phénotype déficient respiratoire peut être désavantageux dans le cadre des procédés de production de levures industrielles.

De manière avantageuse, ce troisième milieu est un milieu pauvre ou minimum, contenant comme seule source de carbone une source de carbone qui ne peut être utilisée que par les cellules qui ont conservé des mitochondries fonctionnelles. On parle dans ce cas de source de carbone respiratoire stricte, c'est à dire de source de carbone impliquant de manière systématique une oxydation mitochondriale et ne produisant pas d'éthanol. Il peut s'agir avantageusement du glycérol ou éventuellement de l'éthanol. En d'autres termes, un tel milieu est dépourvu de sucre fermentescible. Avantageusement, la concentration en glycérol du milieu de croissance est celle couramment mise en œuvre lorsque celui-ci est utilisé comme seule source de carbone, à savoir comprise entre 5 et 50 g/L, avantageusement comprise entre 10 et 50 g/L, par exemple égale à 10 g/L de manière à obtenir une biomasse suffisante pour inoculer le premier milieu de culture du cycle suivant.

Par définition, un milieu minimum contient, outre une source de carbone, une source d'azote, une source de potassium, une source de phosphore, une source de soufre, une source de magnésium, une source de calcium, une source de fer, une source d'oligoéléments et de l'eau.

Un milieu pouvant servir à l'élaboration de ce troisième milieu de culture peut comprendre :
- une base, telle que « yeast nitrogen base » de DIFCO^{®}, avantageusement à hauteur de 3,4 g/L ;
- et éventuellement du sulfate d'ammonium, avantageusement à hauteur de 5 g/L.

Outre la composition spécifique de ce troisième milieu de croissance, la culture de la souche de levure ou du mélange de souches est avantageusement réalisée dans des conditions standard favorables à la croissance des levures, à savoir :
- un pH acide, avantageusement compris entre 4 et 6, voire 4,5 et 5,5, par exemple égal à 5 ;
- une température comprise entre 28 et 37°C, voire entre 30 et 35°C, avantageusement égale à 30°C ;
- sous agitation moyenne, par exemple égale à 150 rpm ;
- en aérobiose. En pratique, la culture peut être réalisée dans une fiole à baffles obturée par un bouchon poreux qui permet l'apport d'O₂ dans le milieu.

Là encore, la culture est stoppée lorsque la source de carbone, avantageusement le glycérol, a été totalement consommée. Il est à noter qu'en pratique, la croissance dans ce milieu est *a priori* moins rapide que dans le second milieu en raison de la source d'azote minérale. Ainsi et de manière avantageuse, la culture est menée pendant plusieurs heures, avantageusement 48 heures.

Comme déjà mentionné, le procédé selon l'invention se caractérise en ce que la culture de la souche de levure, successivement dans les deux ou trois milieux de croissance décrits ci-dessus, est itérée sous forme de cycles.

Le nombre de cycle est choisi de manière à favoriser la sélection de la souche présentant la caractéristique recherchée, à savoir une capacité améliorée à fermenter un pentose en présence d'un acide organique inhibiteur, tout en limitant le nombre de cycles pour éviter l'apparition de tares chez les levures.

Ainsi et de manière avantageuse, le nombre de cycles est au moins égal à deux et avantageusement inférieur ou égal à 10, par exemple égal à 8. En d'autres termes, la culture successive dans au moins les deux milieux de croissance tels que définis est répétée au moins deux fois, avantageusement plus de 2 fois mais moins de 10 fois, par exemple 8 fois.

Selon un autre mode de réalisation avantageux du procédé selon l'invention, la culture successive dans au moins les deux milieux de croissance est répétée au moins deux fois, en présence de concentrations croissantes en acide organique, avantageusement de l'acide acétique, sous forme non dissociée. En d'autres termes, le procédé selon l'invention est réalisé en présence d'au moins deux concentrations distinctes, la première concentration étant inférieure à la seconde. L'augmentation de cette concentration peut se faire de manière graduelle, éventuellement par paliers. Ainsi et de manière avantageuse pour l'adaptation des souches de levure, au moins les deux premiers cycles sont réalisés à concentration constante.

Selon l'invention, la concentration du premier milieu de croissance en acide organique, avantageusement en acide acétique, sous forme non dissociée est comprise entre 1,3 et 2,6 g/L.

Lorsque le nombre de cycles souhaité est atteint et pour obtenir des clones différenciés, la culture est avantageusement étalée sur un milieu solide, encore plus avantageusement sur un milieu synthétique favorable à la croissance des levures, tel que le milieu YPG. Le niveau d'ensemencement est typiquement de 2000 cellules par plaque et la croissance a lieu à 30°C pendant environ 72 heures.

Dans un mode de réalisation particulier, les performances des clones ainsi isolés sont testées.

A cette fin, des précultures en milieu liquide, avantageusement dans un milieu synthétique favorable à la croissance des levures, tel que le milieu YPG, peuvent être réalisées.

De manière avantageuse, ces précultures servent à l'ensemencement d'un milieu de sélection, à savoir un milieu équivalent au premier milieu de croissance décrit ci-dessus, comprenant comme seule source de carbone ledit pentose, avantageusement du xylose, et ledit acide organique, avantageusement de l'acide acétique, sous forme non dissociée. De manière privilégie, une concentration moyenne en acide acétique est choisie, par exemple égale à 5 g/L pour un milieu à pH 5.

De manière avantageuse, les clones sélectionnés sont ceux présentant la meilleure croissance dans ce milieu. Le niveau de croissance peut être déterminé par l'analyse de la turbidité via une mesure de la densité optique à l'aide d'un spectrophotomètre à une longueur d'onde comprise entre 600 et 700 nm, par exemple égale à 630 nm. En pratique, la croissance peut être évaluée dans des microplaques de type DeepWeel ou en milieu liquide dans les conditions énoncées ci-dessous, à savoir :
- un pH acide, avantageusement compris entre 4 et 6, voire 4,5 et 5,5, encore plus avantageusement égal à 5 ;
- une température comprise entre 28 et 37°C, voire entre 30 et 35°C, avantageusement égale à 32°C ;
- sous faible agitation, par exemple égale à 100 rpm, ou sans agitation ;
- dans des conditions réduites d'apport en oxygène (sous O₂ limité ou en anaérobie) ;
- un temps de croissance d'au moins 24 heures, par exemple de 72 heures.

Le taux d'ensemencement doit être modéré pour éviter un effet « titrant » des levures, par exemple égal à 0,25 g/kg eq MS (MS = matière sèche).

Un premier critère important est donc la capacité de la ou des souches de levures sélectionnées à croitre dans un milieu contenant comme seule source de carbone un pentose, en particulier le xylose, en présence d'un inhibiteur de type acide organique sous forme non dissociée, en particulier l'acide acétique.

Selon un autre mode de réalisation, le procédé de sélection peut comprendre une étape d'évaluation de la perte de masse en fonction du temps de fermentation dans le milieu de sélection et dans les conditions tels que définis ci-dessus. La mesure de la perte de masse de la fiole de fermentation est corrélée à la production d'alcool. Le suivi de cette perte de masse reflète donc la cinétique de fermentation du pentose, avantageusement le xylose, en présence d'un inhibiteur de type acide organique sous forme non dissociée, en particulier l'acide acétique.

Dans ces conditions et en présence du seul pentose comme source de carbone (pas de glucose), le profil attendu est monophasique, avec une cinétique associée de type ax + b lorsque le sucre est en concentration non limitante (au lancement de la culture). Dans le cadre de la sélection d'une souche avantageuse, notamment dans sa vitesse de fermentation des pentoses, en particulier du xylose, la valeur a (nombre positif) est aussi élevée que possible.

Par ailleurs, il est recherché une souche de levure capable de résister à l'acide acétique en milieu xylose, et ce y compris à des concentrations élevées en acide acétique. Ainsi, il peut être mesuré la vitesse maximale de production de CO₂ lors de la fermentation du xylose sur un milieu comme décrit précédemment mais contenant des concentrations variables en acide acétique du milieu de fermentation.

Pour une concentration donnée en acide acétique, il est privilégié que la souche sélectionnée présente une vitesse maximale de consommation du xylose, égale au double de la perte de masse en fermentation, plus importante que les souches déjà isolées, et ce quelle que soit la concentration en acide acétique (y compris en l'absence d'acide acétique), avantageusement dans la gamme de concentrations des milieux réels, par exemple entre 4 et 8 g/kg.

Par ailleurs et selon un autre mode de réalisation avantageux, il est vérifié que la capacité de la souche sélectionnée à fermenter le glucose (hexose) et le xylose (pentose) en présence de l'acide organique sous forme non dissociée, avantageusement l'acide acétique, n'est pas affectée. Il s'agit donc là d'analyser les caractéristiques de la fermentation en général, dans un milieu proche des milieux réels contenant à la fois des hexoses et des pentoses.

En pratique, la fermentation est réalisée sur un milieu riche de type YFP, contenant du glucose, du xylose et de l'acide acétique, par exemple un milieu comprenant :
- extrait de levure, avantageusement à hauteur de 10 g/L ;
- bacto-peptone, avantageusement à hauteur de 10 g/L ;
- glucose, avantageusement à hauteur de 55 g/L ;
- xylose, avantageusement à hauteur de 45 g/L ;
- acide acétique, avantageusement avec une quantité introduite dans le milieu à hauteur de 5 g/L ;
- KOH.

Les conditions de culture sont celles adaptées à la croissance et à la fermentation des levures, avantageusement :
- un pH acide, avantageusement compris entre 4 et 6, encore plus avantageusement égal à 5 ;
- une température comprise entre 28 et 37°C, voire entre 30 et 35°C, avantageusement égale à 32°C ;
- sous faible agitation, par exemple égale à 100 rpm ;
- dans des conditions réduites d'apport en oxygène (sous O₂ limité ou en anaérobie) ;
- avec un temps de croissance d'au moins 24 heures, par exemple de 72 heures.

Le taux d'ensemencement doit être modéré pour éviter un effet « titrant » des levures, par exemple égal à 0,25 g/kg eq MS (MS = matière sèche).

Il est attendu un profil illustrant le double effet de l'acide acétique sur les levures aptes à fermenter le xylose et le glucose :
- Pendant la première phase, appelée « phase glucose », un retard d'initiation de la fermentation aussi réduit que possible, avantageusement inférieur à 24 heures, encore plus avantageusement de l'ordre de 5 à 6 heures. De manière privilégiée, la souche sélectionné n'a donc pas perdu de résistance vis-à-vis de l'acide acétique lors de la fermentation du glucose ;
- Lors de la fermentation du xylose, une cinétique aussi favorable que possible, c'est-à-dire aussi rapide et importante que possible, avec des performances meilleures que les souches de l'art antérieur.

Selon un mode de réalisation avantageux, le procédé selon l'invention est mis en œuvre sur une souche de levures présentant des propriétés intéressantes dans le cadre de l'application visée :
- une bonne croissance dans les milieux synthétiques et naturels ;
- une capacité à fermenter le glucose y compris, de manière avantageuse, en présence d'un acide organique sous forme non dissociée, en particulier d"acide acétique ;
- une capacité à fermenter le xylose y compris, de manière avantageuse, en présence d'un acide organique sous forme non dissociée, en particulier d'acide acétique ;
- et avantageusement une inhibition réduite de la voie de fermentation du glucose par l'acide acétique.

De telles souches sont par exemple choisies dans le groupe suivant :
- souche déposée à la CNCM en date du 5.10.2011 sous le numéro 1-4538 ;
- souche déposée à la CNCM en date du 16.05.2013 sous le numéro I-4749 ;
- souche déposée à la CNCM en date du 12.12.2013 sous le numéro I-4829 ;
- souche déposée à la CNCM en date du 9.04.2015 sous le numéro I-4966.

Des souches privilégiées sont la souche déposée à la CNCM en date du 12.12.2013 sous le numéro I-4829 et/ou la souche déposée à la CNCM en date du 9.04.2015 sous le numéro I-4966.

De préférence, les souches sélectionnées à l'issue du procédé selon l'invention conservent les caractéristiques d'intérêt des souches utilisées pour la mise en œuvre dudit procédé.

Selon un autre aspect, la présente invention concerne une souche de levure obtenue à l'aide du procédé décrit ci-dessus, à savoir la souche déposée auprès de la CNCM (Collection Nationale de Cultures de Microorganismes, Institut Pasteur, 25 rue du Docteur Roux, 75724 Paris Cedex 15) sous le numéro I-4953 en date du 29 Janvier 2015.

De manière remarquable, une telle souche présente une capacité à fermenter le xylose en présence d'acide acétique encore jamais égalée.

Cette capacité est avantageusement mesurée dans les conditions suivantes :
Une préculture de la souche est réalisée en milieu liquide, avantageusement dans un milieu synthétique favorable à la croissance des levures, tel que le milieu YPG, et dans les conditions décrites en rapport avec le second milieu mis en œuvre dans le procédé décrit ci-dessus.

Après 24 à 72 heures de culture à 30°C, la souche est transférée dans un milieu de croissance tel que le premier milieu de croissance du procédé selon l'invention. Un tel milieu est avantageusement défini comme suit :
- extrait de levure (EXL), avantageusement à hauteur de 5 g/L ;
- di-amonium phosphate, avantageusement à hauteur de 4,7 g/L ;
- acide citrique, avantageusement à hauteur de 11,4 g/L ;
- citrate tri-sodique, avantageusement à hauteur de 13,5 g/L ;
- ZnSO₄, avantageusement à hauteur de 21,2 mg/L ;
- MgSO₄ 7H₂O, avantageusement à hauteur de 1 g/L ;
- Thiamine, avantageusement à hauteur de 18,24 mg/L ;
- Pyridoxine, avantageusement à hauteur de 5,28 mg/L ;
- Biotine, avantageusement à hauteur de 1,76 g/L ;
- Panthoténate, avantageusement à hauteur de 3,8 mg/L ;
- acide nitotinique, avantageusement à hauteur de 20 mg/L ;
- myo-inositol, avantageusement à hauteur de 50 mg/L ;
- riboflavine, avantageusement à hauteur de 1 mg/L ;
- para-amino-benzoate, avantageusement à hauteur de 1,2 mg/L ;
- tween 80, avantageusement à hauteur de 1 g/L.

Il contient en outre du xylose en tant que seule source de carbone, à une concentration avantageusement égale à 50 g/L et des concentrations variables en acide acétique allant préférentiellement de 0 à 10 g/L de manière à pouvoir établir une courbe effet/dose. Il s'agit ici de valeurs de quantités d'acide acétique introduites dans le milieu de culture à pH 5.

Les conditions d'ensemencement et de culture sont avantageusement les suivantes :
- un pH acide, avantageusement compris entre 4 et 6, voire 4,5 et 5,5, encore plus avantageusement égal à 5 ;
- une température comprise entre 28 et 37°C, voire entre 30 et 35°C, avantageusement égale à 32°C ;
- sous faible agitation, par exemple égale à 100 rpm;
- dans des conditions réduites d'apport en oxygène (sous O₂ limité ou en anaérobie) ;
- un temps de croissance d'au moins 24 heures, avantageusement de 72 heures ;
- avec un taux d'ensemencement avantageusement égal à 0,25 g/kg eq MS (MS = matière sèche).

A l'issue de cette culture, la vitesse maximale de production de CO₂ est déterminée pour chaque concentration en acide acétique.

Dans ces conditions, il a été rapporté pour la première fois la sélection de souches de levures présentant la caractéristique suivante, jamais obtenue par les souches de l'art antérieur : La dose d'acide acétique nécessaire pour réduire de 50% sa cinétique de consommation du xylose est supérieure à 2,5 g/L en milieu liquide (2,5 g/kg en milieu solide), voire 3 g/L, voire supérieure ou égale à 3,5 g/L, avantageusement supérieure ou égale à 4 g/L, voire même supérieure ou égale à 5 g/L. Il s'agit ici de valeurs de quantités d'acide acétique introduites dans le milieu de culture à pH 5.

En pratique, il en résulte une cinétique de fermentation du xylose plus rapide en milieux réels, comprenant typiquement entre 2 et 8 g/kg d'acide acétique. Ainsi et en présence d'une souche selon l'invention, il est possible d'envisager une fermentation totale des sucres en présence en moins de 72 heures, avantageusement entre 48 et 72 heures.

La souche de levure visée par l'invention comprend au moins une copie d'un gène exogène codant la xylose isomérase, avantageusement de *Clostridium phytofermentans.* Dans le cadre de l'invention, on entend par « exogène » le fait que le gène provient d'un autre organisme.

La souche de levure visée par l'invention comprend au moins une copie surnuméraire du gène *GAL2* codant un transporteur des hexoses également capable d'assurer la capture du xylose. Selon un autre mode de réalisation, ladite souche comprend au moins deux copies surnuméraires du gène *GAL2.* Celui-ci peut être placé sous le contrôle d'un promoteur fort et constitutif de type pADH1.

La souche de levure visée par l'invention présente une ou plusieurs caractéristiques choisies dans le groupe suivant :
- la suppression de l'activité aldose réductase (GRE3) ;
- la surproduction de la xylulokinase (XKS1), notamment par modification du promoteur ou introduction de copies surnuméraires ;
- l'expression ou la surproduction de la voie des pentoses phosphates (RPE1, RKl1, TKL1, TAL1, ...) ;
- l'absence d'activité xylose réductase (XR) ;
- la capacité à fermenter l'arabinose, avantageusement grâce à l'introduction de voie bactérienne telle que décrit dans WO 2008/041840 ou EP 1 499 708.

La souche de levure visée par la présente invention appartient au groupe des hemiascomycètes, au genre *Saccharomyces.* Il s'agit d'une souche de *Saccharomyces cerevisiae.*

Dans le cadre de l'invention, on entend par « souche de levure » une population de levures rigoureusement identique du point de vue génétique. Cela englobe aussi bien les souches dites de laboratoire que les souches dites industrielles.

Selon un autre aspect, la présente invention vise également une levure obtenue par culture d'une souche telle que définie ci-dessus.

Dans le cadre de l'invention, on entend par « levure » un produit commercial obtenu grâce à la mise en œuvre d'un procédé de production d'une souche de levure. Ainsi, des levures présentant des caractéristiques différentes peuvent être obtenues à partir d'une même souche, ces différences étant liées au procédé de production mis en œuvre.

Selon un autre aspect, l'invention concerne l'utilisation d'une souche ou d'une levure telle que définie ci-dessus pour la fermentation d'un matériau, contenant avantageusement du xylose et/ou du glucose, et/ou pour la production d'éthanol. Selon un mode de réalisation particulier, le matériau est un matériau lignocellulosique. Un tel matériau contient typiquement :
- des pentoses, en particulier D-xylose et L-arabinose ;
- des hexoses, en particulier du D-mannose, du D-galactose, du L-rhamnose et du D-glucose ;
- des acides uroniques.

Selon un aspect particulier, l'invention a trait à un procédé de production de produits de fermentation ou d'éthanol comprenant les étapes suivantes :
- Incubation d'un matériau ou milieu contenant du xylose avec une souche ou une levure telle que définie ci-dessus ;
- Fermentation en condition anérobie ou semi-anaérobie ;
- Récupération du ou des produits de fermentation, ou de l'éthanol.

Selon un mode de réalisation particulier de ce procédé, le matériau ou milieu contient également du glucose.

La présente invention va être illustrée plus avant à l'aide des exemples de réalisation qui suivent, à l'appui des figures annexées. Toutefois, ceux-ci n'ont aucune portée limitative.

### LEGENDES DES FIGURES :

La figure 1 représente l'évolution de la perte de masse (exprimée en g/kg) en fonction du temps de fermentation de différentes souches, dans un milieu contenant du xylose à 50 g/L et de l'acide acétique à 5 g/L à pH = 5.
La figure 2 représente l'impact de la concentration en acide acétique (exprimée en g/L à pH 5) sur la vitesse de perte de masse (production du CO₂ à partir du xylose), lors de la fermentation de différentes souches, dans un milieu contenant du xylose à 50 g/L.
La figure 3 représente l'évolution de la perte de masse (exprimée en g/kg) en fonction du temps de fermentation de différentes souches, dans un milieu contenant du glucose à 55 g/L, du xylose à 45 g/L et de l'acide acétique à 5 g/L (à pH 5).

### EXEMPLES DE REALISATION

### I/ Matériel et Méthodes :

### 1/ Souche :

Levure : *Saccharomyces cerevisiae*
Souche : I-4829 ou I-4966

### 2/ Milieux et conditions de culture :

### 2-a/ Evolution dirigée :

Milieu 1: YFX50 =
- xylose 50 g/L ;
- extrait de levure (EXL) 5 g/L ;
- di-amonium phosphate 4,7 g/L ;
- acide citrique 11,4 g/L ;
- citrate tri-sodique 13,5 g/L ;
- ZnSO₄ 21,2 mg/L ;
- MgSO₄ 7H₂O 1 g/L ;
- thiamine 18,24 mg/L ;
- pyridoxine 5,28 mg/L ;
- biotine 1,76 g/L ;
- panthoténate 3,8 mg/L ;
- acide nitotinique 20 mg/L ;
- myo-inositol 50 mg/L ;
- riboflavine 1 mg/L ;
- para-amino-benzoate 1,2 mg/L ;
- tween 80 1 g/L.

Les levures sont cultivées dans ce milieu avec le xylose comme seule source de carbone, et de l'acide acétique dont la concentration augmente au cours des cycles (voir section Résultats).

Le pH du milieu est maintenu à 5.

La culture est réalisée à 32°C sous une agitation de 100 rpm dans des fioles obturées par des bouchons qui permettent de réduire l'apport d'oxygène dans le milieu et de laisser échapper le CO₂ en surpression qui est produit tout au long de cette culture. La culture dure environ 7 jours dans ces conditions.

### Milieu 2 (YPG) :

- 10 g/L d'extrait de levure ;
- 10 g/L de peptone ;
- 20 g/L de glucose comme seule source de carbone.

La culture est réalisée à 30°C sous une agitation à 150 rpm dans des fioles à baffles obturées par des bouchons poreux qui permettent l'apport d'oxygène dans le milieu. La culture dure environ 24 heures dans ces conditions.

### Milieu 3 :

- 3,4 g / L de « yeast nitrogen base » DIFCO^{®} ;
- 5 g / L sulfate d'ammonium ;
- 10 g/ L de glycérol comme seule source de carbone.

La culture est réalisée à 30°C sous une agitation à 150 rpm dans des fioles à baffles obturées par des bouchons poreux qui permettent l'apport d'oxygène dans le milieu. La culture dure environ 24 à 48 heures dans ces conditions.

### 2-b/ Sélection des souches les plus performantes :

A l'issue du 8^{ème} cycle, les cellules ont été étalées à hauteur de 2000 cellules par boite (150 mm de diamètre) de milieu gélosé (YPG + agar).

Les colonies ainsi obtenues ont ensuite été cultivées en format Deep Well (= microplaque à 96 puits) dans du milieu YPG.

Après 72 heures de culture à 30°C, les colonies ont été transférées dans du milieu YFX50-Ac-5000 dont la composition est indiquée ci-dessous :

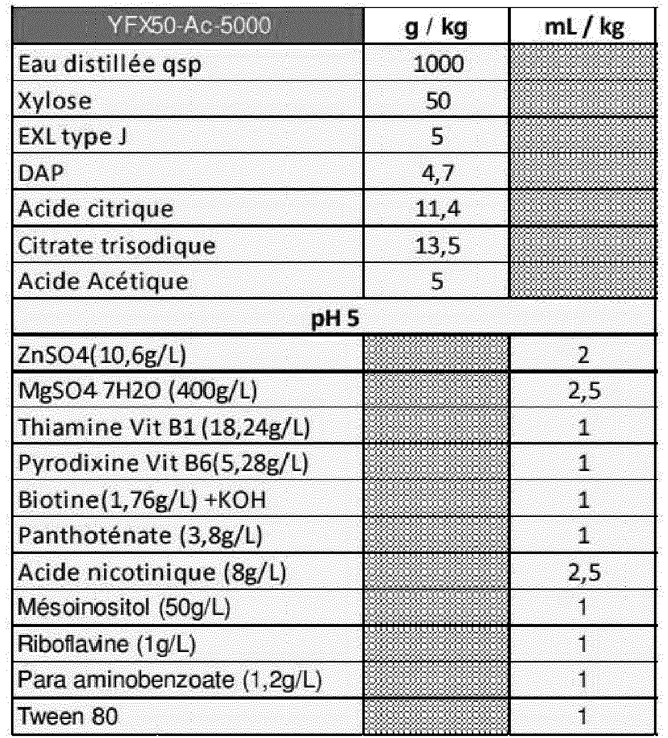

En pratique, il s'agit du même milieu de culture que le premier milieu de croissance utilisé dans le procédé selon l'invention, avec une concentration en acide acétique (quantité introduite dans le milieu de culture à pH 5) égale à 5 g/L.

De même, les conditions de culture sont similaires :

### Conditions :

pH: 5
Température : 32 °C
Conditions en O₂ : sans apport d'oxygène (anaérobie)
Agitation : 100 rpm
Durée de la culture : 72 heures
Préculture / Incubation :
   - Reprise des colonies pour les cultures liquides en Deep Well (microplaques à 96 puits) ;
   - 0,25 g/kg eq MS pour les cultures liquides.

### 2-c/ Validation des souches isolées :

Milieu : YFP-5000
- 10 g/L d'extrait de levure ;
- 10 g/L de bacto-peptone ;
- 55 g/L de glucose;
- 45 g/L de xylose;
- 5 g/L d'acide acétique (quantité introduite dans le milieu de culture à pH 5);
- KOH.

### Conditions:

pH: 5
Température : 32°C
Conditions en O₂ : sans apport d'oxygène
Agitation : 100 rpm
Durée de la culture : jusqu'à 72 heures
Préculture / Incubation : 0,25 g/kg eq MS des levures préalablement propagées en milieu YPG.

### 3/ Evaluation de la perte de masse :

La production d'éthanol est mesurée de manière indirecte par une mesure de la perte de masse de la fiole de fermentation, cette perte de masse étant directement corrélée à la production d'alcool. Elle est exprimée en g par kg de milieu.

### 4/ Evaluation de la vitesse de production du CO₂ :

A partir de la détermination de la perte de masse (correspondant à la production de CO₂), il est possible de déterminer la vitesse de consommation du xylose (exprimée en g/L) qui est le double de la perte de masse.

La vitesse maximale de production de CO₂ est exprimée en g.kg⁻¹.h⁻¹.g⁻¹ de MS (matière sèche).

### II/ Résultats :

### 1/ Sélection des souches d'intérêt :

### 1-a/ Evolution dirigée :

L'évolution dirigée a été réalisée en mode SBR (« Single Batch Repeat ») :
- Cycles 1 à 3 : milieu 1 à 3 g/L en acide acétique (ajouté dans le milieu à pH 5) puis milieu 2 puis milieu 3 ;
- Cycles 4 à 6 : milieu 1 à 4 g/L en acide acétique puis milieu 2 puis milieu 3 ;
- Cycle 7 : milieu 1 à 5 g/L en acide acétique puis milieu 2 puis milieu 3 ;
- Cycle 8 : milieu 1 à 6 g/L en acide acétique puis milieu 2 puis milieu 3.

### 1-b/ Sélection des souches les plus performantes :

Après isolement des clones sur milieu solide et leur préculture en milieu liquide, celles-ci sont ensemencées dans des microplaques de type Deep Well contenant du milieu YFX50-Ac-5000 (50 g/kL de xylose et 5 g/L d'acide acétique à pH = 5).

Après 72 heures de fermentation dans ce milieu, les souches sélectionnées (16 au total) sont celles présentant la meilleure croissance, par analyse de la densité cellulaire du milieu. Les souches CNCM I-4071 et CNCM I-4829 ont servi respectivement de témoin négatif et positif en regard de la capacité des cellules à utiliser le xylose pour faire leur croissance.

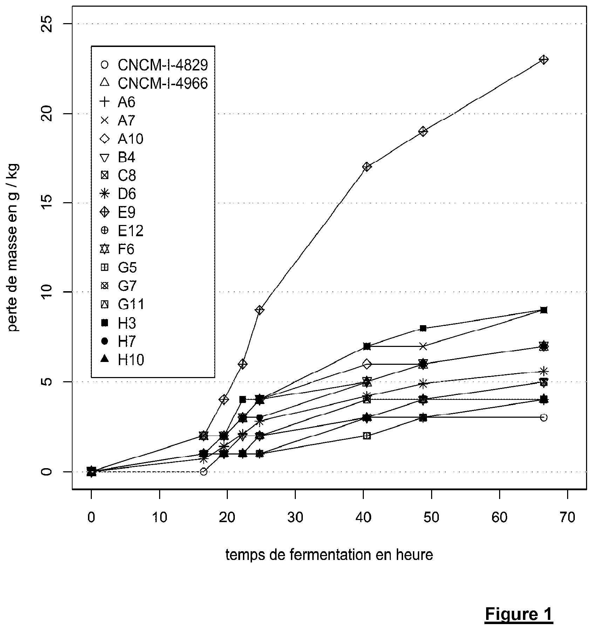

Ces 16 souches ont été évaluées d'un point de vue cinétique sur ce même milieu. Les résultats sont présentés à la Figure 1, qui représente la perte de masse en fonction du temps de fermentation. De manière attendue dans la mesure où ce milieu contient uniquement du xylose (pas de glucose), le profil observé pour toutes les souches testées est monophasique. En revanche, il apparaît que la dérivée à l'origine de la courbe varie en fonction des souches. Celle-ci peut être formulée à l'aide de l'équation ax + b, avec a qui varie selon les souches.

La figure 1 révèle que la souche E9 présente les meilleures performances. Cette souche a été déposée auprès de l'Institut Pasteur (Collection Nationale de Cultures de Microorganismes, 25 rue du Docteur Roux, 75724 Paris Cedex 15) sous le numéro CNCM I-4953 en date du 29 janvier 2015 et a été utilisée dans la suite des expériences.

### 2/ Propriétés de la souche 1-4953 :

### 2-1/ En milieu xylose :

La première étape a consisté à déterminer l'impact de l'évolution dirigée sur la capacité de cette souche à résister à l'acide acétique lors de la fermentation du xylose. Pour cela, le test a consisté à mesurer la vitesse maximale de production de CO₂ lors de la fermentation du xylose sur un milieu YFX50-Ac. Afin d'obtenir une courbe effet/dose, différentes concentrations en acide acétique ont été ajoutées dans le milieu de fermentation.

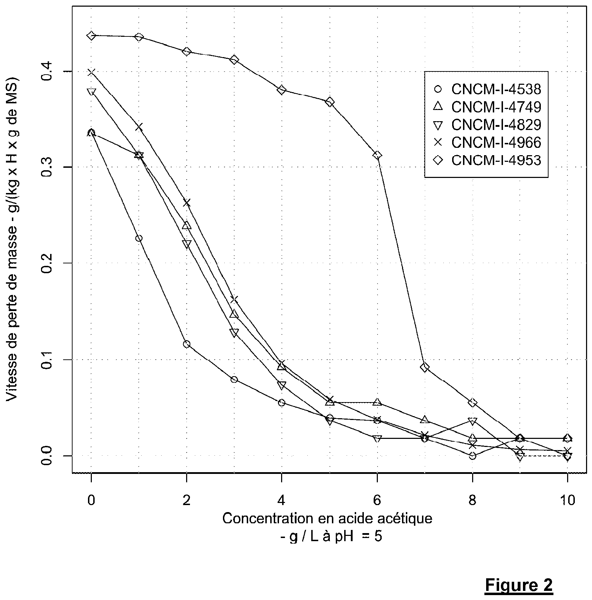

La figure 2 montre que cette souche a une vitesse maximale de consommation du xylose, égale au double de la perte de masse en fermentation, qui est environ 15% plus importante que celle de la souche I-4829 en absence d'acide acétique. Cet écart est encore plus important quand la dose d'acide acétique est de 4 g/L. En effet, à cette concentration, la vitesse de consommation du xylose est réduite de 85% pour la souche I-4829, contre seulement 15% pour la souche I-4953. Cela signifie qu'en présence d'acide acétique à 4 g/L et à pH 5, la souche I-4953 a la même vitesse de consommation du xylose que la souche I-4829 en absence d'acide acétique.

### 2-2/ En milieu glucose + xylose :

Afin de mieux appréhender l'impact de l'évolution dirigée sur la fermentation en générale, la perte de masse en fonction du temps de fermentation a été suivie sur un milieu contenant du glucose, du xylose et de l'acide acétique.

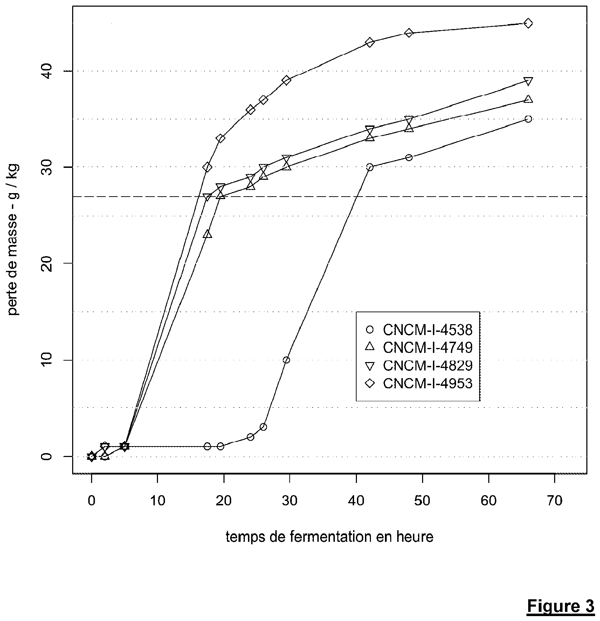

Les résultats sont présentés à la figure 3, qui illustre clairement le double effet de l'acide acétique sur les levures aptes à fermenter le xylose :
- Pendant la première phase, appelée « phase glucose », la souche I-4538 présente un retard d'initiation de la fermentation d'environ 24 heures. Pour les autres souches (I-4749, I-4829 et I-4953), le lag n'est que de 5 à 6 heures. Ce constat signifie que la souche isolée n'a pas perdu de résistance vis-à-vis de l'acide acétique lors de la fermentation du glucose ;
- Lorsque la fermentation du xylose prend le relais, les cinétiques des souches I-4538, I-4749 et I-4829 sont comparables. En revanche, pour la souche I-4953, il est notable que la production de CO₂ (et par conséquent celle d'éthanol) est plus rapide et importante que celle observée pour les 3 autres souches.

Ces résultats confirment l'obtention d'une souche plus résistante vis-à-vis de l'acide acétique lors de la fermentation du xylose, et donc globalement plus performante.

### III/ Conclusions :

Le procédé décrit a donc permis de sélectionner au moins une souche stable, I-4953, qui a conservé sa capacité à fermenter le glucose avec un retard d'initiation limité, tout en présentant une cinétique de fermentation du xylose en présence d'acide acétique améliorée.

## Revendications

1. Procédé de sélection d'une souche de levure présentant une capacité améliorée à fermenter un pentose, avantageusement le xylose, en présence d'acide organique, avantageusement d'acide acétique, sous forme non dissociée,
dans lequel au moins une souche de levure présentant une capacité à fermenter ledit pentose est cultivée successivement dans les 2 milieux suivants :
- un premier milieu de croissance comprenant comme seule source de carbone ledit pentose et ledit acide organique sous forme non dissociée ;
- un deuxième milieu de croissance comprenant comme seule source de carbone une autre source de carbone, avantageusement du glucose, et dépourvu dudit acide organique sous forme non dissociée,
la culture successive dans au moins ces deux milieux de croissance étant répétée au moins deux fois, en présence de concentrations croissantes en acide organique sous forme non dissociée à une concentration comprise entre 1,3 et 2,6 g/L.

2. Procédé de sélection d'une souche de levure selon la revendication 1, ***caractérisé* en ce que**, à chaque cycle ou au moins une fois dans le procédé, après la culture dans les deux premiers milieux de croissance, une culture est réalisée en aérobiose dans un milieu minimum contenant comme seule source de carbone une source de carbone respiratoire stricte, avantageusement du glycérol.

3. Procédé de sélection d'une souche de levure selon la revendication 1 ou 2, ***caractérisé* en ce que** la culture de la souche, successivement dans les deux ou trois milieux de croissance, est répétée plus de 2 fois mais moins de 10 fois, avantageusement 8 fois.

4. Procédé de sélection d'une souche de levure selon l'une des revendications précédentes, ***caractérisé* en ce que** la souche de levure présentant une capacité à métaboliser ledit pentose est choisie dans le groupe suivant : I-4538, I-4749, I-4829 et I-4966, avantageusement I-4829 et/ou I-4966.

5. Souche de levure susceptible d'être obtenue à l'aide du procédé selon l'une des revendications 1 à 4 déposée auprès de la CNCM (Collection Nationale de Cultures de Microorganismes, Institut Pasteur, 25 rue du Docteur Roux, 75724 Paris Cedex 15) sous le numéro I-4953 en date du 29 Janvier 2015.

6. Levure obtenue par culture d'une souche selon la revendication 5.

7. Utilisation d'une souche selon la revendication 5 ou d'une levure selon la revendication 6 pour la fermentation d'un matériau, contenant avantageusement du xylose, et/ou pour la production d'éthanol.

8. Procédé de production de produits de fermentation ou d'éthanol comprenant les étapes suivantes :
- Incubation d'un matériau ou milieu contenant du xylose avec une souche selon la revendication 5 ou une levure selon la revendication 6 ;
- Fermentation en condition anaérobie ou semi-anaérobie ;
- Récupération du ou des produits de fermentation, ou de l'éthanol.

9. Procédé de production de produits de fermentation ou d'éthanol selon la revendication 8, ***caractérisé* en ce que** le matériau ou milieu contient également du glucose.

## Patentansprüche

1. Verfahren zur Auswahl eines Hefestamms, der eine verbesserte Fähigkeit zur Fermentierung einer Pentose, vorzugsweise Xylose, in Anwesenheit organischer Säure, vorzugsweise Essigsäure, in nicht dissoziierter Form, hat
bei dem mindestens ein Hefestamm, der die Fähigkeit zur Fermentierung dieser Pentose hat, nacheinander kultiviert wird in den folgenden 2 Milieus:
- ein erstes Wachstumsmilieu, das als einzige Kohlenstoffquelle diese Pentose und diese organische Säure in nicht dissoziierter Form enthält;
- ein zweites Wachstumsmilieu, das als einzige Kohlenstoffquelle eine andere Kohlenstoffquelle enthält, vorteilhafterweise Glucose, und ohne die erwähnte organische Säure in nicht dissoziierter Form,
die aufeinanderfolgende Kultur in mindestens diesen beiden Wachstumsmilieus wird mindestens zwei Mal wiederholt, in Anwesenheit steigender Kozentrationen an organischer Säure in nicht dissoziierter Form bei einer Konzentration zwischen 1,3 und 2,6 g/L.

2. Verfahren zur Auswahl eines Hefestamms nach Anspruch 1, ***dadurch gekennzeichnet, dass*** bei jedem Zyklus oder zumindest einmal im Verfahren, nach der Kultivierung in den beiden ersten Wachstumsmilieus, eine Kultur aerob durchgeführt wird, in einem minimalen Milieu, das als einzige Kohlenstoffquelle eine strikte Atemgas- Kohlenstoffquelle enthält, vorzugsweise Glycerol.

3. Verfahren zur Auswahl eines Hefestamms nach Anspruch 1 oder 2, ***dadurch gekennzeichnet, dass*** die Kultur des Stamms, nacheinander in den zwei oder drei Wachstumsmilieus, mehr als 2 Mal wiederholt wird, aber weniger als 10 Mal, vorteilhafterweise 8 Mal.

4. Verfahren zur Auswahl eines Hefestamms nach einem der vorangehenden Ansprüche ***dadurch gekennzeichnet, dass*** der Hefestamm, der die Fähigkeit zur Metabolisierung dieser Pentose ausgewählt wird aus der folgenden Gruppe: I-4538, I-4749, I-4829 und I-4966, vorteilhafterweise I-4829 und/ oder I-4966.

5. Hefestamm, der mit Hilfe des Verfahrens nach einem der Ansprüche 1 bis 4 erzeugt werden kann, registriert bei der CNCM (Collection Nationale de Cultures de Microorganismes, Institut Pasteur, 25 rue du Docteur Roux, 75724 Paris Cedex 15) unter der Nummer I-4953, mit Datum vom 29. Januar 2015.

6. Hefe, hergestellt durch Kultur eines Stamms nach Anspruch 5.

7. Einsatz eines Stamms nach Anspruch 5 oder einer Hefe nach Anspruch 6 zur Fermentierung eines Materials, das vorteilhafterweise Xylose enthält und/ oder zur Erzeugung von Ethanol.

8. Verfahren zur Erzeugung von Fermentierungs- oder Ethanolprodukten, das die folgenden Schritte enthält:
- Inkubation eines Xylose enthaltenden Materials oder Milieus mit einem Stamm nach Anspruch 5 oder einer Hefe nach Anspruch 6;
- Fermentierung unter anaeroben oder halb-anaeroben Bedingungen;
- Auffangen des oder der Fermentierungsprodukte oder des Ethanols.

9. Herstellungsverfahren für Fermentierungsprodukte oder Ethanol. nach Anspruch 8, ***dadurch gekennzeichnet, dass*** das Material oder Milieu auch Glucose enthält.

## Claims

1. A method for selecting a yeast strain having improved capability for fermenting a pentose, advantageously xylose, in the presence of organic acid, advantageously acetic acid, in non-dissociated form,
in which at least one yeast strain that is capable of fermenting said pentose is consecutively cultured in the following two media:
- a first growth medium comprising said pentose as the only carbon source and said organic acid in non-dissociated form;
- a second growth medium comprising another carbon source as the only carbon source, advantageously glucose, free of said organic acid in non-dissociated form;
the consecutive culture in at least said two growth media being repeated at least twice, in the presence of rising concentrations of organic acid in non-dissociated form at a concentration between 1.3 and 2.6 g/L.

2. The method for selecting a yeast strain according to claim 1, ***characterized* in that**, after the culture in the two first growth media or at least once in the method, a culture is done in aerobiosis in a minimum medium containing as the only carbon source a source of strict respiratory carbon, advantageously glycerol.

3. The method for selecting a yeast strain according to claim 1 or 2, ***characterized* in that** the culture of the strain, consecutively in the two or three growth media, is repeated more than two times but less than 10 times, advantageously eight times.

4. The method for selecting a yeast strain according to one of the preceding claims, ***characterized* in that** the yeast strain having a capability to metabolize said pentose is chosen from the following group: 1-4538, 1-4749, 1-4829 and 1-4966, advantageously I-4829 and/or 1-4966.

5. A yeast strain which is obtainable by the method according to one of claims 1 to 4 ***characterized* in that** it was deposited at the CNCM (Collection Nationale de Cultures de Microorganismes, Institut Pasteur, 25 rue du Docteur Roux, 75724 Paris Cedex 15) under number 1-4953 on January 29, 2015.

6. A yeast obtained by culture of a strain according to claim 5.

7. Use of a strain according to claim 5 or a yeast according to claim 6 for fermentation of a material, advantageously containing xylose, and/or for the production of ethanol.

8. A production method for fermentation products or ethanol comprising the following steps:
- Incubation of a material or medium containing xylose with a strain according to claim 5 or a yeast according to claim 6;
- Fermentation under anaerobic or semi-anaerobic conditions;
- Recovery of the one or more fermentation products, or ethanol.

9. The production method for fermentation products or ethanol according to claim 8, ***characterized* in that** the material or medium also contains glucose.
